# NEUE EUROPÄISCHE PATENTSCHRIFT

(11) **EP 0 784 972 B2**
(45) Veröffentlichungstag und Bekanntmachung des Hinweises auf die Entscheidung über den Einspruch: **18.03.2015**
(45) Hinweis auf die Patenterteilung: 28.07.2010
(21) Anmeldenummer: 96120023.5
(22) Anmeldetag: 13.12.1996
(51) Int. Cl.: A61K 8/60, A61Q 19/00

(54) **Kosmetische und/oder pharmazeutische Emulsionen**
Cosmetic and/or pharmaceutical emulsions
Emulsions cosmétiques et/ou pharmaceutiques

(30) Priorität: 22.12.1995 DE 19548345
(43) Veröffentlichungstag der Anmeldung: 23.07.1997
(73) Patentinhaber: Cognis IP Management GmbH, 40589 Düsseldorf (DE)
(72) Erfinder: Ansmann, Achim, Dr., 40699 Erkrath (DE); Kawa, Rolf, 40789 Monheim (DE); Fabry, Bernd, Dr., 41352 Korschenbroich (DE)

(56) Entgegenhaltungen:
- EP-A1- 0 662 318
- WO-A-93/11865
- WO-A-96/28245
- WO-A1-92/06778
- WO-A1-92/07543
- WO-A1-93/00065
- WO-A1-94/16668
- WO-A1-94/16677
- DE-A- 4 243 272
- DE-A- 4 409 321
- DE-A- 19 524 210
- US-A- 5 214 030
- US-A- 5 266 690
- CHEMICAL ABSTRACTS, vol. 122, no. 22, 29.Mai 1995 Columbus, Ohio, US; abstract no. 268690, AOI, NOBUYUKI ET AL: "Emulsifying to solubilizing agents" XP002037958 & JP 07 008 781 A (TAIYO KAGAKU KK, JAPAN)

## Beschreibung

### Gebiet der Erfindung

Die Erfindung betrifft kosmetische und pharmazeutische Emulsionen mit verminderter Viskosität in der lamellaren Gelphase, enthaltend ausgewählte Emulgatormischungen und Ölkörper vom Typ der Guerbetalkohole.

### Stand der Technik

Zur Herstellung kosmetischer und pharmazeutischer Mittel, wie beispielsweise Cremes, Lotionen oder Salben, werden Öle und Wasser eingesetzt, die unter Einsatz geeigneter Emulgatoren zu einer Emulsion verarbeitet werden. Insbesondere dann, wenn diese Emulsionen hydrophile Wachse wie beispielsweise Partialglyceride oder Fettalkohole enthalten, wird bei der Herstellung eine lamellare Gelphase durchlaufen, die eine hohe Viskosität besitzt und eine ausreichende Durchmischung erschwert. In der Folge kann es vorkommen, daß die Produkte trotz intensiven Rührens keine ausreichende Homogenität besitzen und unkontrolliert in der Viskosität ansteigen.

Aus der Europäischen Patentschrift EP-B1 0 553 241 (SEPPIC) ist die Verwendung von Mischungen aus Alkyloligoglucosiden, Fettalkoholen und gegebenenfalls Polyglucose zur Herstellung von Emulsionen bekannt. Auch gemäß der Lehre der internationalen Patentanmeldung WO 92/07543 (Henkel) lassen sich Alkyloligoglucoside zusammen mit Fettalkoholen und Partialglyceriden als kosmetische Emulgatoren einsetzen. Beide Schriften bieten für das Problem der unerwünschten Gelphasenbildung keine Lösung an. Dazu beschreibt Patentanmeldung WO 94/16677 (Henkel) eine Haarzubereitung mit Alkyloligoglucoside und 2-0ctyldodecanol.

Die Aufgabe der vorliegenden Erfindung hat nun darin bestanden, das Problem der Bildung hochviskoser Gele bei der Herstellung von Emulsionen zuverlässig zu verhindern und Emulsionen mit einer konstant niedrigen Viskosität zur Verfügung zu stellen.

### Beschreibung der Erfindung

Gegenstand der Erfindung sind kosmetische und/oder pharmazeutische Emulsionen, enthaltend
(a) Alkyloligoglucoside,
(b2) Polyolpoly-12-hydroxystearate und ggf. (b1) Fettalkohole
   der allgemeinen Formel (II) R²OH, in der R2 für einen
   aliphatischen, linearen Alkylrest mit 8 bis 22 Kohlenstoffatomen
   steht und
(c) Guerbetalkohole, wobei das Gewichtsverhältnis Emulgator (a+b) zu Ölkörper 1 : 10 bis 1 : 1 beträgt.

Aus umfangreichen Untersuchungen hat die Anmelderin gefunden, daß sich das Problem der Bildung hochviskoser lamellarer Gele zufriedenstellend lösen kann, wenn man Emulgator und Ölkörper entsprechend aufeinander abstimmt. So wurde überraschenderweise gefunden, daß Emulsionen wie beansprucht besonders niedrigviskose Gelphasen bilden.

### Alkyloligoglucoside

Alkyloligoglucoside stellen bekannte nichtionische Tenside dar, die der Formel **(1)** folgen,

**R¹O-[G]ₚ** **(1)**

in der R1 für einen Alkylrest mit 8 bis 22 Kohlenstoffatomen, G für einen Glucoserest und p für Zahlen von 1 bis 10 steht. Sie können nach den einschlägigen Verfahren der präparativen organischen Chemie erhalten werden. Stellvertretend für das umfangreiche Schrifttum sei hier auf die Schriften EP-A1-0301298 und WO 90/03977 verwiesen. Die Indexzahl p in der allgemeinen Formel **(1)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglucosid eine analytisch ermittelte rechnerische Größe, die meistens eine gebrochene Zahl darstellt. Vorzugsweise werden Alkyloligoglucoside mit einem mittleren Oligomerisierungsgrad p von 1,1 bis 3,0 eingesetzt. Aus anwendungstechnischer Sicht sind solche Alkyloligoglucoside bevorzugt, deren Oligomerisierungsgrad kleiner als 1,7 ist und insbesondere zwischen 1,2 und 1,4 liegt. Der Alkylrest R¹ kann sich von primären Alkoholen mit 8 bis 22 Kohlenstoffen ableiten. Typische Beispiele sind Alkyloligoglucoside auf Basis von Caprylalkohol, Caprinalkohol, Laurylalkohol, Myristylalkohol, Cetylalkohol, Stearylalkohol, Isostearylalkohol und/oder Behenylalkohol sowie deren technischen Gemischen. Vorzugsweise werden Alkyloligoglucoside eingesetzt, die sich von Fettalkoholen mit 8 bis 16,12 bis 14, 12 bis 16 und insbesondere 16 bis 18 Kohlenstoffatomen ableiten.

### Fettalkohole

Unter Fettalkoholen sind primäre aliphatische Alkohole der Formel (II) zu verstehen,

**R²OH** **(II)**

in der R2 für einen aliphatischen, linearen Alkylrest mit 8 bis 22 Kohlenstoffatomen steht. Typische Beispiele sind Caprylalkohol, Caprinalkohol, Laurylalkohol, Myristylalkohol, Cetylalkohol, Stearylalkohol, und Behenylalkohol sowie deren technische Mischungen, die z.B. bei der Hochdruckhydrierung von technischen Methylestern auf Basis von Fetten und Ölen anfallen. Bevorzugt sind technische Gemische mit 16 bis 18 kohlenstoff. atomen wie insbesondere Cetearylalkohol. Im Sinne der Erfindung ist es besonders vorteilhaft, Mischungen von Alky-loligoglucosiden und Fettalkoholen einzusetzen, die identische Alkylreste aufweisen, also beispielsweise Mischungen von Cetearyloligoglucosiden und Cetearylalkohol.

### Polyolpoly-12-hydroxystearate

Bei den Polydpdy-12-hydroxystearaten handelt es sich um bekannte Stoffe, die beispielsweise unter der Handelsbezeichnung "Dehymuls® PGPH" von der Henkel KGaA, Düsseldorf/FRG vertrieben werden.

Die Polyolkomponente der Emulgatoren kann sich von Stoffen ableiten, die über mindestens zwei, vorzugsweise 3 bis 12 und insbesondere 3 bis 8 Hydroxylgruppen und 2 bis 12 Kohlenstoffatome verfügen. Typische Beispiele sind:
- Glycerin und Polyglycerin;
- Alkylenglycole, wie beispielsweise Ethylenglycol, Diethylenglycol, Propylenglycol;
- Methyolverbindungen, wie insbesondere Trimethylolethan, Trimethylolpropan, Trimethylolbutan, Pentaerythrit und Dipentaerythrit;
- Alkyloligoglucoside mit 1 bis 22, vorzugsweise 1 bis 8 und insbesondere 1 bis 4 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit.
- Zucker mit 5 bis 12 Kohlenstoffatomen, wie beispielsweise Glucose oder Saccharose;
- Aminozucker wie beispielsweise Glucamin.

Unter den erfindungsgemäß einzusetzenden Emulgatoren kommt Umsetzungsprodukten auf Basis von Polyglycerin wegen ihrer ausgezeichneten anwendungstechnischen Eigenschaften eine besondere Bedeutung zu. Als besonders vorteilhaft hat sich die Verwendung von ausgewählten Polyglycerinen erwiesen, die die folgende Homologenverteilung aufweisen (in Klammern angegeben sind die bevorzugten Bereiche):

| | |
|---|---|
| Glycerin: | 5 bis 35 (15 bis 30) Gew.-% |
| Diglycerin: | 15 bis 40 (20 bis 32) Gew.-% |
| Triglycerine: | 10 bis 35 (15 bis 25) Gew.-% |
| Tetraglycerine: | 5 bis 20 (8 bis 15) Gew.-% |
| Pentaglycerine: | 2 bis 10 (3 bis 8) Gew.-% |
| Oligoglycerine: | ad 100 Gew.-% |

Das Gewichtsverhältnis zwischen den Alkyloligoglucosiden der Emulgatorkomponente (a) einerseits und den Polyolpoly-12-hydroxystearaten und ggf. den Fettalkoholen andererseits, die die Emulgatorkomponente (b) ausmachen, kann im Bereich 5 : 95 : 60 : 40 liegen und beträgt vorzugsweise 10 : 90 bis 50 : 50.

### Guerbetalkohole

Guerbetalkohole stellen bekannte Ölkörper dar, die man üblicherweise durch basenkatalysierte Kondensation von Fettalkoholen herstellt. Eine Übersicht zu dem Thema ist von A.J.O'Lennick und R.E.Bilbo in **Soap Cosm. Chem. Spec. April, 52 (1987)** erschienen. Bevorzugte Guerbetalkohole leiten sich von Fettalkoholen mit 6 bis 22 Kohlenstoffatomen ab. Guerbetalkohole, die als Bestandteil von Emulsionen eine langanhaltende Hautglättung vermitteln und daher bevorzugt sind, werden auf Basis von C₆-C₁₂-Fettalkoholmischungen hergestellt, enthaltend Capronalkohol, Caprylalkohol, 2-Ethylhexanol, Caprinalkohol und/ oder Laurylalkohol. Ein typischer Fettalkoholschnitt, der als Ausgangsstoff für die bevorzugten Guerbetalkohole in Frage kommt, enthält weniger als 5 Gew.% C₆, 50 bis 60 Gew.-% C₈, 35 bis 45 Gew.% C₁₀ und weniger als 2 Gew.% C₁₂. Guerbetalkohole dieser Art sind in der Deutschen Patentschrift DE-C1 43 41 794 (Henkel) näher beschrieben, deren Lehre ausdrücklich miteinbezogen wird. Das Gewichtsverhältnis Emulgator (a+b) zu Ölkörper beträgt 1 : 10 bis 1 : 1 und liegt vorzugsweise im Bereich 1 : 5 bis 1 : 3.

### Gewerbliche Anwendbarkeit

Die erfindungsgemäßen Emulsionen zeichnen sich in der Gelphase durch eine besonders niedrige Viskosität aus. Sie eignen sich daher zur Herstellung von kosmetischen bzw. pharmazeutischen Zubereitungen wie beispielsweise Tagescremes, Nachtcremes, Pflegecremes, Nährcreme, Bodylotions, Salben und dergleichen. Als weitere Hilfs- und Zusatzstoffe können Tenside, weitere Ölkörper, Co-Emulgatoren, kationische Polymere, Siliconöle, Überfettungsmittel, Fette, Wachse, Stabilisatoren, biogene Wirkstoffe, Glycerin, Konservierungsmittel, Verdickungsmittel, Farb- und Duftstoffe enthalten sein.

### Tenside

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether)phosphate. Sofern die anionischen. Tenside Polyglycoletherketten enthalten, können diese eine konventionelle, vorzugsweise jedoch eine eingeengte Homologenverteilung aufweisen.

Typische Beispiele für **nichtionische Tenside** sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöl-additive", Thieme Verlag, Stuttgart,1978, S.123-217** verwiesen.

### Ölkörper

Als zusätzliche Ölkörper kommen beispielsweise Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimerdiol) und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate, Dialkylether und/oder aliphatische bzw. naphthenische Kohlenwasserstoffe in Betracht.

### Co-Emulgatoren

Als nichtionogene **O/W-Co-Emulgatoren** kommen in Betracht (a1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe (a2) C_{12/18}-Fettsäuremono- und -diester von Anlagerungsprodukten von 1 bis 30 Mol Ethylenoxid an Glycerin; (a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte; (a4) Anlagerungsprodukte von 15 bis 60 Mol Ethylenoxid an Ricinusöl und/oder gehärtetes Ricinusöl. Ebenfalls geeignet sind Gemische von Verbindungen aus mehreren dieser Substanzklassen. Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus DE-PS 20 24 051 als Rückfettungsmittel für kosmetische Zubereitungen bekannt.

Als **W/O-Co-Emulgatoren** kommen in Betracht: (b1) Anlagerungsprodukte von 2 bis 15 Mol Ethylenoxid an Ricinusöl und/oder gehärtetes Ricinusöl; (b2) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose); (b3) Trialkylphosphate; (b4) Wollwachsalkohole; (b5) Polysiloxan-Polyalkyl-Polyether-Copolymere bzw. entsprechende Derivate; (b6) Mischester aus Pentaerythrit, Fettsäuren, Citronensäure und Fettalkohol gemäß DE-PS 11 65 574 sowie (b7) Polyalkylenglycole.

Geeignete **kationische Polymere** sind beispielsweise kationische Cellulosederivate, kationische Stärke, Copolymere von Diallylammoniumsalze und Acrylamide, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Proteinpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der FR-A 22 52 840 sowie deren vernetzte wasserlöslichen Polymere, kationische Chitinderivate wie beispielsweise quaterniertes Chitosan, gegebenenfalls mikrokristallin verteilt, kationischer Guar-Gum wie z.B. Jaguar® CBS, Jaguar® C-17, Jaguar® C-16 der Celanese/US sowie quaternierte Ammoniumsalz-Polymere wie z.B. Mirapol® A-15, Mirapol® AD-1 und Mirapol® AZ-1 der Miranol/US.

Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Stabilisatoren** können Metallsalze von Fettsäuren wie z.B. Magnesium-, Aluminium und/oder Zinkstearat eingesetzt werden. Unter **biogenen Wirkstoffen** sind beispielsweise Pflanzenextrakte und Vitaminkomplexe zu verstehen. Als **Konservierungsmittel** eignen sich beispielsweise Phenoxyethanol, Formaldehydlösung, Parabene, Pentandiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.%, bezogen auf die gesamte Mischung, eingesetzt.

Typische Emulsionen weisen Zusammensetzungen gemäß Tabelle 1 auf (mit der Maßgabe, daß sich die Angaben zu 100 Gew.-% ergänzen).

**Tabelle 1**

| **Typische Zusammensetzung von Lotionen und Cremes** | | |
|---|---|---|
| **Bestandteil** | **Lotion [Gew.-%]** | **Creme [Gew.-%]** |
| Alkylpolyglucoside | 1 bis 2 | 1 bis 3 |
| Fettalkohole | 2 bis 4 | 4 bis 7 |
| Polyolpol-12-hydroacystearat | 1 bis 2 | 1 bis 2 |
| Guerbetalkohole | 10 bis 20 | 10 bis 20 |
| Hilfs- und Zusatzstoffe | 1 bis 5 | 1 bis 5 |
| Wasser | ad 100 | |

Der Gesamtanteil der Hilfs- und Zusatzstoffe kann 1 bis 10, vorzugsweise 2 bis 5 Gew.-% - bezogen auf die Mittel - betragen. Die Herstellung der Mittel kann in an sich bekannter Weise, d.h. beispielsweise durch Heiß-, Heiß-Heiß/Kalt- bzw. PIT-Emulgierung erfolgen. Hierbei handelt es sich um ein rein mechanisches Verfahren, eine chemische Reaktion findet nicht statt.

### Beispiele

Die Herstellung der Emulsionen erfolgte im Heißverfahren bei 80°C. Die Rezepturen R3 und R4 sind erfindungsgemäß, die Rezepturen R1 und R2 sowie R5 bis R10 dienen zum Vergleich. Die Viskosität der Proben wurde nach der Brookfield-Methode
in einem RVF-Viskosimeter (10 UpM, Spindel 5) sofort sowie nach Lagerung über 7 Tage bei 20 bzw. 40°C bestimmt. Die Ergebnisse sind in Tabelle 2 zusammengefaßt.

### Eingesetzte Substanzen (CTFA-Nomenklatur)

A1) Hexadecyl Polyglucose
A2) Hexadecyl Alcohol
A3) Polyglyceryl Poly-12-Hydroxystearate
B1) Octyldodecanol
B2) Dicaprylyl Ether
B3) Decyl Oleate
B4) Almond Oil

**Tabelle 2**

| **Viskosität und Lagerstabilität (Mengenangaben als Gew.-%, Wasser ad 100 %)** | | | | | | | | | | |
|---|---|---|---|---|---|---|---|---|---|---|
| **Komponenten** | **R1** | **R2** | **R3** | **R4** | **R5** | **R6** | **R7** | **R8** | **R9** | **R10** |
| A1 | 1,9 | 2,25 | 1,9 | 2,25 | 1,9 | 1,9 | 2,25 | 2,25 | 1,9 | 1,9 |
| A2 | 3,1 | 2,25 | 3,1 | 2,25 | 3,1 | 3,1 | 2,25 | 2,25 | 3,1 | 3,1 |
| A3 | - | - | 1,2 | 1,2 | - | 1,2 | - | - | - | - |
| B1 | 20 | 16 | 20 | 16 | - | - | - | - | - | - |
| B2 | - | - | - | - | 20 | 20 | - | - | - | - |
| B3 | - | - | - | - | - | - | 16 | - | 20 | - |
| B4 | - | - | - | - | 16 | - | - | - | - | 20 |

| *Viskosität [mPas]* | | | | | | | | | | |
|---|---|---|---|---|---|---|---|---|---|---|
| *- sofort* | 8.000 | 8.000 | 10.000 | 10.000 | 13.600 | 14.000 | 12.000 | 12.000 | 14.600 | 14.800 |
| *- 7d, 20°C* | 8.000 | 8.000 | 10.000 | 9.800 | 18.000 | 19.500 | 15.600 | 16.600 | 20.400 | 20.400 |
| *- 7d, 40°C* | 8.000 | 8.000 | 9.800 | 10.000 | 22.600 | 28.000 | 19.600 | 19.000 | 24.400 | 26.800 |

Man erkennt, daß bei Einsatz der erfindungsgemäßen Emulgatorcompounds zusammen mit Guerbetalkoholen niedrigviskose, lagerstabile Emulsionen gebildet werden. Zusammen mit anderen Ölkörpern werden höherviskose Produkte erhalten, deren Viskosität nicht stabil ist, sondern im Laufe der Lagerung insbesondere bei höheren Temperaturen zunimmt.

## Patentansprüche

1. Kosmetische und/oder pharmazeutische Emulsionen, enthaltend
(a) Alkyloligoglucoside,
(b2) Polyolpoly-12-hydroxystearate und ggf. (b1) Fettalkohole der allgemeinen Formel (II) R²OH in der R2 für einen aliphatischen, linearen Alkylrest mit 8 bis 22 Kohlenstoffatomen steht und
(c) Guerbetalkohole
wobei das Gewichtsverhältnis von Emulgator (a +b) zu Ölkörper 1 :10 bis 1:1 beträgt

2. Emulsionen nach Anspruch 1, **dadurch gekennzeichnet, dass** sie Alkyloligoglucoside der Formel (1) enthalten,
*R¹O-[G]ₚ* *(1)*
in der R1 für einen linearen, gesättigten Alkylrest mit 8 bis 22 Kohlenstoffatomen, G für einen Glucoserest und p für eine Zahl im Bereich von 1 bis 10 steht.

3. Emulsionen nach den Ansprüchen 1 bis 2, **dadurch gekennzeichnet, dass** sie Polyglycerinpoly-12-hydroxystearate enthalten.

4. Emulsionen nach den Ansprüchen 1 bis 3, **dadurch gekennzeichnet, dass** sie Guerbet-alkohole auf Basis von Fettalkoholen mit 6 bis 22 Kohlenstoffatomen enthalten.

## Claims

1. Cosmetic and/or pharmaceutical emulsions containing
(a) alkyl oligoglucosides
(b2) polyol poly-12-hydroxystearates and optionally (b1) fatty alcohols corresponding to general formula (II) R²OH in which R² is an aliphatic, linear alkyl radical containing 8 to 22 carbon atoms and
(c) Guerbet alcohols
wherein the weight ratio of emulsifier (a+b) to oily body is 1:10 to 1:1.

2. Emulsions as claimed in claim 1, **characterized in that** they contain alkyl oligoglucosides corresponding to formula (I):
R¹O-[G]ₚ (I)
in which R¹ is a linear, saturated alkyl radical containing 8 to 22 carbon atoms, G is a glucose radical and p is a number of 1 to 10.

3. Emulsions as claimed in claims 1 to 2, **characterized in that** they contain polyglycerol poly-12-hydroxystearates.

4. Emulsions as claimed in claims 1 to 3, **characterized in that** they contain Guerbet alcohols based on fatty alcohols containing 6 to 22 carbon atoms.

## Revendications

1. Emulsions cosmétiques et/ou pharmaceutiques, contenant
(a) des alkyloligoglucosides
(b2) des polyolpoly-12-hydroxystéarates et le cas échéant (b1) des alcools gras de formule générale (II) R²OH dans laquelle R² représente un radical alkyle aliphatique, linéaire comprenant 8 à 22 atomes de carbone, et
(c) des alcools de Guerbet
où le rapport pondéral d'émulsifiant (a + b) à corps huileux est de 1:10 à 1:1.

2. Emulsions selon la revendication 1, **caractérisées en ce qu'**elles contiennent des alkyloligoglucosides de formule (I),
R¹O-[G]ₚ (I)
dans laquelle
R¹ représente un radical alkyle saturé, linéaire comprenant 8 à 22 atomes de carbone,
G représente un reste de glucose et
p représente un nombre dans la plage de 1 à 10.

3. Emulsions selon les revendications 1 à 2, **caractérisées**
**en ce qu'**elles contiennent des polyglycérolpoly-12-hydroxystéarates.

4. Emulsions selon les revendications 1 à 3, **caractérisées**
**en ce qu'**elles contiennent des alcools de Guerbet à base d'alcools gras comprenant 6 à 22 atomes de carbone.
